# EUROPEAN PATENT APPLICATION

(11) **EP 1 362 588 A1**
(43) Date of publication of application: **19.11.2003**
(21) Application number: 02703857.9
(22) Date of filing: 20.02.2002
(51) Int. Cl.: A61K 31/196, A61K 31/5575, A61K 9/20, A61K 9/28, A61K 9/48, A61K 9/16, A61K 47/14, A61K 47/38, A61P 43/00, A61P 29/00, A61P 19/02, A61P 19/00, A61P 21/00, A61P 25/06, A61P 15/00, A61P 19/06, A61P 13/10, A61P 27/02

(54) **MEDICINAL COMPOSITIONS COMPRISING DICLOFENAC AND ORNOPROSTIL**

(30) Priority: 22.02.2001 JP 2001046430
(71) Applicant: ONO PHARMACEUTICAL CO., LTD., Osaka-shi, Osaka 541-8526 (JP)
(72) Inventor: SAKAI, Yoshiki, c/o Ono Pharmaceutical Co., Ltd, Mishima-gun, Osaka 618-8585 (JP); KATSUBE, Nobuo, c/o Ono Pharmaceutical Co., Ltd, Mishima-gun, Osaka 618-8585 (JP); NISHIURA, Akio, c/o Ono Pharmaceutical Co., Ltd, Mishima-gun, Osaka 618-8585 (JP)
(74) Representative: Henkel, Feiler, Hänzel
(86) International application number: JP0201474
(87) International publication number: WO02066030

(57) **Abstract**

Medicinal compositions which contain Diclofenac or the nontoxic salt and Ornoprostil. The medicinal composition comprising the combination of the Diclofenac or the nontoxic salt and Ornoprostil can be expect on the immediate effect compared with commercial Diclofenac tablets and Arthrotec tablets, has only a little side reaction (especially, gastrointestinal ulcer, diarrhoea/vomitus, and nephropathy), and excellent antipyretic, paregoric, and antiinflammatory effect.

## Description

### TECHNICAL FIELD

The present invention relates to a medicinal composition which comprises Diclofenac or its nontoxic salt and Ornoprostil.

### BACKGROUND OF THE INVENTION

So far, non-steroidal antiinflammatory drugs (Non-Steroidal AntiInflammatory Drug; hereafter, abbreviated to NSAID) are known to be effective to rheumatoid arthritis, osteoarthritis, and backache, etc. as antipyretic drugs, analgesic drugs, and antiphlogistines, and a lot of compounds have been marketed up to now. It is said that there are these action mechanisms in NSAID in inhibition of the cyclooxygenase enzyme (COXI and COXII are known) which is PGH2 synthesis route from arachidonic acid and repression of various prostaglandins biosynthesis (for example, PGE2, PGl2, PGFr2A, TXA2, and PGD2, etc.). As typical NSAID, for example, there are Diclofenac sodium (Diclofenac sodium: sodium o-(2,6-dichloroanilino)-phenylacetate), indomethacin (Indomethacin: 1 -(p-chlorobenzoyl)-5-methoxy-2-methylindol-3-acetic acid), acemetacin (Acemetacin: 2-[2-[1-(p-chlorobenzoyl)-5-methoxy-2-methylindol-3-yl]-acetoxy]acetic acid), etc.

However, because NSAID inhibits prostaglandin biosynthesis (it is chiefly called PGl2 and PGE2) which has a mucosal protective effect in digestive organs, it has serious side effects which cause digestive disturbance (hereafter, it is referred to as digestive disturbance.) such as gastric mucosal disturbance, ulcus ventriculis, and ulcus duodenis, and exaggerates nephropathy because of inhibition of prostaglandin biosynthesis (it is chiefly called PGl2 and PGE2) in the kidney and decrease of the renal blood flow. For digestive disturbance of these side effects, a method of suppressing digestive disturbance by combined medication with attack factor inhibitors such as antiulcer agents, for example, promoters of protection factor such as Teprenone (Teprenone: 3:2(5E:5Z)geometrical mixture of (9E, 13E)-6,10,14,18-tetramethyl-5,9,13,17-nonadecatetraen-2-one), Rebamipide (Rebamipide: (±)-2-(4-chlorobenzoylamino)-3-[2(1H)-quinolinon-4-yl]propionic acid), Ecabet sodium (Ecabet sodium: (+)-(1R,4aS,10aR)-1,2,3,4,4a,9,10,10a-octahydro-1,4a-dimethyl-7-(1-methylethyl)6-sulfo-1-phenanthrencecarboxylic acid 6-sodium salt pentahydrate), etc.; restrainers of acid secretion (proton pump inhibitor (PPl), for example, Lansoprazole (Lansoprazole: (±)-2-[[[3-methyl-4-(2,2,2-trifluoroethoxy)-2-pyridyl]methyl]sulfinyl]benzimidazole) and Omeprazole (Omeprazole: (±)-5-methoxy-2-[[(4-methoxy-3,5-dimethyl-2-pyridyl)methyl]sulfinyl]benzimidazole), etc.), H2 blocker, for example, ranitidine hydrochloride (Ranitidine hydrochloride: N-[2-[[5-[(dimethylamino) methyl]furfuryl]thio)ethyl]-N'-roethyl-2-nitro-1,1-ethenediamine hydrochloride) or famotidine (Famotidine: 3-[[[2-[(diaminomethylene)amino]-4-thiazolyl]-methyl]thio]-N²-sulfamoylpropionamidine), etc.; or misoprostol described later, etc. is adopted.

### BACKGROUND ART

Prostaglandins (hereafter, abbreviated to PG) are physiologically active substances secreted from various tissues of organism. These exhibit strong actions by a little over wide range such as an increased action of vascular flow, an inhibitory action of platelet aggregation, an inhibitory action of gastric acid secretion, a protection action of gastric mucosa, and an oxytocic action, etc., while these have a fault that are metabolized in a short time. Therefore, a lot of stable derivatives are synthesized, and some have been already sold as antiulcer drugs. For example, 3 compounds, which are shown by formula (I), 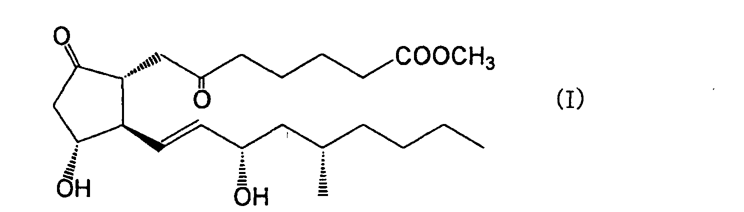 17S, 20-dimethyl-6-keto PGE1 methyl ester (the generic name: Ornoprostil (Ornoprostil); hereafter, it is referred to as Ornoprostil), shown by formula (A), 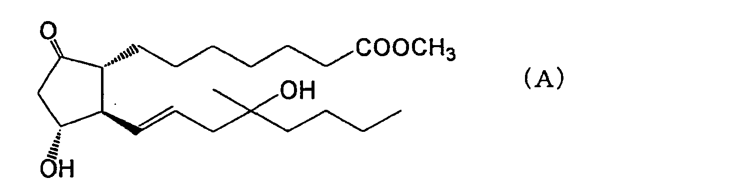 15-deoxy-16-hydroxy-16-methyl-PGE1 methyl ester the generic name: Misoprostol (Misoprostol); hereafter, it is referred to as Misoprostol), and shown by formula (B), 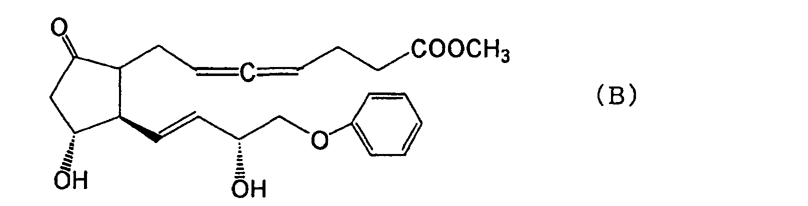 16-Phenoxy-17, 18, 16-19, 20-tetranor-Δ4-PGE2 methyl ester (the generic name: Enprostil (Enprostil)) have been marketed.

So far, it has been tried to use the PG kind to reduce digestive disturbance by NSAID. For example, it has been reported that combined medication of indomethacin and Ornoprostil, or Acemetacin and Ornoprostil could reduced digestive disturbance compared with single administration of NSAID (reference; *Progress in Medicine,* 9(7), 183-190 (1989)). Moreover, a mixture (Arthrotec tablets: trade name) which contains Diclofenac sodium and Misoprostol, has been developed recently, and has already been marketed in Europe and America. However, since the combinations of such NSAID and the PG kind does not have enough antipyretic, analgesic, antiinflammatory effect or a reductive action of digestive disturbance (diarrhoea, tummy ache, abdominal full-consciousness, maldigestion, vomiturition, and vomitus, etc.) and generated side effects of PG, it can't be said the satisfactory combination. On a package insert of the above mixture, it has been described that for example, in administering the above mixture which contains Diclofenac sodium and Misoprostol, side effects (diarrhoea, tummy ache, abdominal full-consciousness, maldigestion, vomiturition, and vomitus, etc.) of PG have frequently happened compared with a single administration of Diclofenac sodium.

### DISCLOSURE OF THE INVENTION

Therefore, there are subjects of the present invention in finding the combinations of NSAID and PG that can make the best use of the action (antipyretic, analgesic, antiinflammatory effect) of NSAID and reduce the side effects (digestive disturbance and nephropathy), then suppress side effects (diarrhoea and vomitus, etc.) of PG.

Considering the above subjects, as a result of zealously examining the most effective combination of NSAID and the PG kind, these inventors found that the combination of Diclofenac, which has the strongest antipyretic, analgesic, and antiinflammatory effect in NSAID, or the nontoxic salt and Ornoprostil could be the best, and completed this invention.

Moreover, as a result of examining administration times of Diclofenac or the nontoxic salt and Ornoprostil, they found that an incidence of digestive disturbance could be minimized without weakening antipyretic, analgesic, and antiinflammatory effect when both were administered simultaneously or Ornoprostil was administered earlier than administration of Diclofenac or the nontoxic salt.

Moreover, they succeeded in manufacturing a single formulation (a compounding ingredient which contains Diclofenac or the nontoxic salt and Ornoprostil in a single formulation), which had an ideal discharge pattern by use of medium chain triglyceride (hereafter, it might be abbreviated to MCT.).

In addition, they first confirmed that Ornoprostil was effective to nephropathy (glycero-induced nephropathy). Therefore, it can be expected that the compounding ingredient of Omoprostil and Diclofenac could reduce exacerbation of nephropathy which is a side reaction of Diclofenac.

That is, the present invention relates to:
1. a medicinal composition comprising Diclofenac or its nontoxic salt and Ornoprostil,
2. the medicinal composition of preceding clause 1, which is an antipyretic, an analgesic drug and/or an antiphlogistine,
3. the medicinal composition of preceding clause 2, which is a medicine and /or prophylactic drug for rheumatoid arthritis, rheumatic fever, osteoarthritis, ankylosing spondylitis, spondylopathy, periarthritis, cervico-omo-brachial syndrome, tendon marrow inflammation, neuralgia, arthralgia, muscle soreness, backache, postoperative, posttraumatic or after tooth extraction pain, inflammation, Sprain pain, bruise pain, toothache, headache, gout pain, algomenorrhea, dysmenorrhea, afterpain, pelvic imflammatory diaease, spine inflammation, urocystitis, anterior segment inflammation and/or acute upper respiratory inflammation.
4. the medicinal composition of preceding clause 2 or 3, which can reduce digestive disturbance induced by Diclofenac or the nontoxic salt,
5. the medicinal composition of preceding clause 4, which can reduce digestive disturbance induced by Diclofenac or the nontoxic salt,
6. a formulation of the medicinal composition of preceding clause 1, wherein Omoprostil is discharged simultaneously with or before discharge of Diclofenac or the nontoxic salt in gastrointestinal tract,
7. the formulation of preceding clause 6, which contains Diclofenac or the nontoxic salt, Ornoprostil, and a stabilizer of Ornoprostil,
8. in addition, the formulation of preceding clause 7, which contains a suspending agent,
9. the formulation of any one of preceding clauses 6 to 8 , which is a soft capsule mixed Diclofenac with Ornoprostil,
10. the formulation of preceding clause 6 or 7, which is a tablet mixed Ornoprostil with Diclofenac,
11. the formulation of preceding clause 6 or 7, which is a tablet coated with Ornoprostil to a nucleus made from Diclofenac,
12. the formulation of preceding clause 6 or 7, which is a hard capsule comprising a soft capsule containing Diclofenac and a solution containing Ornoprostil,
13. the formulation of preceding clause 6 or 7, which is a hard capsule comprising a granule, a capsule or a tablet containing Ornoprostil and a granule, a capsule or a tablet containing Diclofenac,
14. the formulation of preceding clause 7, wherein the stabilizer of Ornoprostil is medium chain triglyceride (MCT),
15. the formulation of preceding clause 14, which is a capsule comprising an MCT solution in which Ornoprostil has been dissolved and Diclofenac or the nontoxic salt has been scattered,
16. the formulation of preceding clause 14, which is a hard capsule comprising a tablet or a capsule which contains an MCT solution containing Ornoprostil and Diclofenac or the nontoxic salt,
17. the formulation of preceding clause 14, which is a hard capsule comprising a capsule which contains an MCT solution containing Ornoprostil and a tablet or a capsule which contains Diclofenac or the nontoxic salt,
18. the formulation of preceding clause 14, which is a hard capsule comprising a capsule which contains an MCT solution containing Ornoprostil and a tablet of Diclofenac or the nontoxic salt, which is covered with coating containing a water-soluble polymer,
19. the formulation of preceding clause 18, wherein the water-soluble polymer is hydroxypropylmethylcellulose,
20. the formulation of any one of preceding clauses 14 to 18, wherein MCT is tricaprilin,
21. a method of reducing digestive disturbance and/or nephropathy induced by Diclofenac or its nontoxic salt, which comprises administering a medicinal composition comprising Ornoprostil or its nontoxic salt,
22. the method of reducing digestive disturbance and/or nephropathy induced by Diclofenac or the nontoxic salt of preceding clause 21, wherein Ornoprostil is administered simultaneously with or before administration of Diclofenac or the nontoxic salt,
23. a prophylactic drug and/or medicine for nephropathy, which comprises Ornoprostil as an active ingredient, and
24. a prophylactic and/or therapeutic method for nephropathy, which comprises administering Ornoprostil.

### BRIEF DESCRIPTION OF THE DRAWINGS

Figure 1 shows the composition of a soft capsule which is an example of mixture of the present invention, which was obtained as Examples 1 and 2 of formulation.
Figure 2 shows the composition of hard capsule-1 which is an example of mixture of the present invention, which was obtained as Example 3 of formulation.
Figure 3 shows the composition of hard capsule-2 which is an example of mixture of the present invention, which was obtained as Example 4 of formulation.

### DETAILED DESCRIPTION OF THE INVENTION

Diclofenac used in the present invention is used as a free carboxylate or its nontoxic salt (for example, alkali salt such as sodium, potassium, and alkaline-earth salts such as magnesium and calcium, etc.), or those mixtures, desirably a compound of a nontoxic salt (especially, sodium salt) or a mixture of the nontoxic salt and the free carboxylate.

Ornoprostil used in the present invention contains Omoprostil itself or the alpha-, beta-, gamma-cyclodextrin inclusion compound, desirably Ornoprostil itself.

The combination of Diclofenac or the nontoxic salt and Ornoprostil means that both may be administered a formulation mixed with a formulation (combination drug) or may be administered formulations made separately (compounding ingredients), desirably combination ingredients. In any case, they are orally administered usually.

Because the medicinal composition (formulation) of the present invention has antipyretic, analgesic, and antiinflammatory effect, it is possible to use them as a medicine and/or a prophylactic drug for disease (for example, rheumatoid arthritis, rheumatic fever, osteoarthritis, ankylosing spondylitis, spondylopathy, periarthritis (periarthritis scapulo-humeralis, etc.), cervico-omo-brachial syndrome, tendon marrow inflammation, neuralgia, arthralgia, muscle soreness, backache, postoperative, posttraumatic or after tooth extraction pain, inflammation, Sprain pain, bruise pain, toothache, headache, gout pain, algomenorrhea, dysmenorrhea, afterpain, pelvic imflammatory diaease, spine inflammation, urocystitis, anterior segment inflammation and/or acute upper respiratory inflammation (cold, common cold, and pharyngolaryngitis, etc.), wherein Diclofenac or the nontoxic salt shows the effect.

Formulations of the present invention can greatly suppress appearance of digestive disturbance and exaggerate nephropathy by shortening discharge time of Ornoprostil than that of Diclofenac or the nontoxic salt (hereafter, especially, unless otherwise indicated, it is only written as Diclofenac.) or making both the same without weakening effectiveness.

That is, in case of combined medication, digestive disturbance and nephropathy by Diclofenac are reduced by administering Ornoprostil before that of Diclofenac or simultaneously. An administering time of Ornoprostil is desirably from 0 minutes (simultaneously with or just before administration) to 60 minutes ago from administration of Diclofenac, more desirably, from 0 minutes (simultaneously or just before administration) to 15 minutes ago, especially desirably from 0 minutes (simultaneously or just before administration) to 10 minutes ago.

In case of compounding ingredients, it is desirable to use what is so prepared as to show such a discharge pattern that Diclofenac is discharged after Ornoprostil is discharged or simultaneously with the discharge.

For example, as compounding ingredients,
1) Soft capsules wherein Ornoprostil was mixed with Diclofenac,
2) Tablets wherein Ornoprostil was mixed with Diclofenac,
3) Tablets coated to a nucleus of Diclofenac by Ornoprostil,
4) Hard capsules which contain soft capsules containing a solution comprising Ornoprostil and Diclofenac,
5) Hard capsule, etc., which contain granules, capsules or tablets containing Omoprostil and granules, capsules or tablets containing Diclofenac, are enumerated.

A stabilizing agent of Omoprostil used in the present invention is medium chain triglyceride (MCT), sugar, sugar alcohol, oligosaccharide, hydroxypropylcellulose, hydroxypropylmethylcellulose, hydroxypropyl starch, hydroxypropylmethylcellulose acetate succinate, hydroxypropylmethylcellulose phthalate, polyvinylpyrrolidone, or methacrylate copolymer, etc.

Medium chain triglyceride (MCT) used in the present invention is what a fatty acid part in the glyceride is caproic acid, caprylic acid, capric acid, or lauric acid, etc., desirably, is tricaprilin, tricaprin, and more desirably is tricaprilin.

Suspending agents used in the present invention are monostearic acid glyceride, hardened oil, or polysorbate 80, etc.

As a water-soluble polymer (used as a coating) used in the present invention, cellulose (hydroxypropylcellulose, hydroxypropylmethylcellulose, and methyl cellulose, etc.), synthetic polymer (polyvinylpyrrolidone, polyvinyl alcohol, and methacrylate copolymer, etc.), and gelatin, etc. are enumerated.

These water-soluble polymers can be used by mixing single or second classes or more. A plasticizer (polyethylene glycol and triethyl citrate, etc.) may be usually included in coating besides water-soluble polymer.

### [Manufacturing methods]

Because Diclofenac and Omoprostil, which are constituents of the medicinal composition of the present invention, are both medicines which have already been marketed, they could be manufactured by well-known methods.

In case of combined medication, a commercial form may be respectively used as it is and a form (tablets, pills, capsules (hard capsules and soft capsules), powders, and granules, etc.) prepared according to a well-known method may be used. For example, by mixing each activator with remedium constituens (lactose, mannitol, glucose, microcrystalline cellulose, starch, etc.), binder pharmaceuticals (hydroxypropylcellulose, (polyvinylpyrrolidone, magnesium aluminometasilicate, etc.), disintegrators (cellulose glycolic acid calcium, etc.), adequate agent (magnesium stearate, etc.), stabilizing agents (medium chain triglyceride, sugar, sugar alcohol), oligosaccharide, hydroxypropylcellulose, hydroxypropylmethylcellulose, hydroxypropyl starch, and hydroxypropyl methylcellulose acetate succinate, hydroxypropylmethylcellulose phthalate, polyvinylpyrrolidone, and methacrylate copolymer, etc), solubilizer (glutamate and aspartate, etc.), and suspending agents (mono stearic acid glyceride, hardened oil, and polysorbate 80, etc.), it is possible to manufacture formulations by the law of the art. Moreover, if necessary, by coating with coating (water-soluble polymer (the cellulose kind (hydroxypropylcellulose, hydroxypropylmethylcellulose, methyl cellulose, hydroxypropylcellulose phthalate), the synthetic polymer kind (polyvinylpyrrolidone, polyvinyl alcohol, methacrylate copolymer, etc.), gelatin, etc.), saccharose, plasticizer ((polyethylene glycol, triethyl citrate, etc.), etc.), an aimed formulation can be obtained.

Compounding ingredients can be manufactured according to methods described as follows, methods described in formulation examples of following descriptions, or well-known methods.

### 1) Manufacturing method of soft capsules which Ornoprostil was mixed with Diclofenac:

Aimed soft capsules can be obtained by making a prescription solution, which is obtained by dissolving and distributing each active constituent with a stabilizing agent (medium chain triglyceride, sugar, sugar alcohol, oligosaccharide, hydroxypropylcellulose, hydroxypropylmethylcellulose, hydroxypropyl starch, hydroxypropyl methylcellulose acetate succinate, hydroxypropylmethylcellulose phthalate, polyvinylpyrrolidone, and methacrylate copolymer, etc.), and adding a suspending agent (mono stearic acid glyceride, hardened oil, and polysorbate 80, etc.), into soft capsules by using soft capsule cover which contains gelatin, glycerin, and sugar alcohol, etc. by the law of the art.

### 2) Manufacturing method of tablets which Ornoprostil was mixed with Diclofenac:

Granules of Ornoprostil can be obtained by adding remedium constituens (hydroxypropylcellulose, hydroxypropylmethylcellulose, hydroxypropyl starch, hydroxypropyl methylcellulose acetate succinate, hydroxypropylmethylcellulose phthalate, polyvinylpyrrolidone, methacrylate copolymer, oligosaccharide, and medium chain triglyceride, etc.) and stabilizing agents (lactose, maltose, mannitol, xylitol, sorbitol, and trehalose, etc.) to Ornoprostil and making it to powder.

Aimed tablets can be obtained by mixing the obtained granule with Diclofenac, remedium constituens (starch, microcrystalline cellulose, lactose, maltose, mannitol, xylitol, sorbitol, and trehalose, etc.), binder pharmaceuticals (hydroxypropylcellulose, hydroxypropylmethylcellulose, polyvinylpyrrolidone, and polyvinyl alcohol), disintegrators (crossing povidone, hydroxypropylcellulose, hydroxypropyl starch, and hydroxypropylmethylcellulose, etc.), lubricant (magnesium stearate etc.), etc. and making it into tablets by the law of the art. Moreover, if necessary, it is also possible to coat them by a coating (water-soluble polymer (cellulose (hydroxypropylcellulose, hydroxypropylmethylcellulose, and methyl cellulose, etc.), synthetic polymers (polyvinylpyrrolidone, polyvinyl alcohol, and methacrylate copolymer, etc.), and gelatins, etc.) and plasticizer (polyethylene glycol and triethyl citrate, etc.) and Opadry, etc.).

### 3) Manufacturing method of tablets which coats Ornoprostil to nucleus of Diclofenac:

By mixing Diclofenac with remedium constituens (starch, microcrystalline cellulose, lactose, maltose, mannitol, xylitol, sorbitol, and trehalose, etc.), binder pharmaceuticals (hydroxypropylcellulose, hydroxypropylmethylcellulose, polyvinylpyrrolidone, and polyvinyl alcohol), disintegrators (crossing povidone, hydroxypropylcellulose, hydroxypropyl starch, and hydroxypropylmethylcellulose, etc.), solubilizer (hydroxypropylmethylcellulose, methacrylate copolymer, polyvinylpyrrolidone, and macrogol, etc.), and lubricant (magnesium stearate etc.), etc., and the mixture is made into tablets according to the law of the art.

Aimed tablets can be obtained by coating the obtained tablets by using stabilizing agents (hydroxypropylcellulose, hydroxypropylmethylcellulose, hydroxypropyl starch, hydroxypropyl methylcellulose acetate succinate, hydroxypropylmethylcellulose phthalate, polyvinylpyrrolidone, methacrylate copolymer, oligosaccharide, and medium chain triglyceride, etc.) and coating (water-soluble polymer (cellulose (hydroxypropylcellulose, hydroxypropylmethylcellulose, and methyl cellulose, etc.), synthetic polymers (polyvinylpyrrolidone, polyvinyl alcohol, and methacrylate copolymer, etc.), and gelatins, etc.) and plasticizer (polyethylene glycol and triethyl citrate, etc.) and Opadry , etc.) with Ornoprostil by the law of the art.

### 4) Manufacturing method of hard capsules which contain soft capsules containing Diclofenac and solution containing Ornoprostil:

Aimed capsules can be obtained by filling a prescription solution in which the soft capsule containing Diclofenac obtained according to the technique described in 1) and Ornoprostil is dissolved and suspended to a stabilizing agent (medium chain triglyceride, sugar, sugar alcohol, oligosaccharide, hydroxypropylcellulose, hydroxypropylmethylcellulose, hydroxypropyl starch, hydroxypropyl methylcellulose acetate succinate, hydroxypropylmethylcellulose phthalate, polyvinylpyrrolidone, and methacrylate copolymer, etc.), into hard capsules (gelatin or hydroxypropylmethylcellulose) and closing a capsule combining site by the law of the art.

### 5) Manufacturing method of hard capsules which contain granules, capsules or tablets containing Ornoprostil and granules, capsules or tablets containing Diclofenac:

Aimed capsules can be obtained by filling soft capsules, granules or tablets of Ornoprostil obtained according to the method described in 1), 2), and 3) and soft capsules, granules or tablets of Diclofenac obtained by similar method into hard capsules (gelatin or hydroxypropylmethylcellulose) by the law of the art.

### [Dosage]

Though dosages of Diclofenac and Omoprostil are different depending on age, weight, symptom, therapeutic effect, medication method, and processing time, etc., usually, as daily dose, each is orally administered within a range from 10 mg to 200 mg and a range from 0.1 µg to 20 µg, respectively, an adult from once to several times a day separately.

Of course, because the dosage of each constituent changes according to various conditions, it might be enough in less amount than the above dosage and might be necessary beyond the range.

### [Pharmacological activity]

The medicinal composition of the present invention is excellent in the drug action of antipyretic, analgesic and antiinflammatory effects, etc. as NSAID, it is possible to use as a medicine and/or a prophylactic medicine against rheumatoid arthritis, rheumatic fever, osteoarthritis, ankylosing spondylitis, spondylopathy, periarthritis (periarthritis scapulo-humeralis, etc.), cervico-omo-brachial syndrome, tendon marrow inflammation, neuralgia, arthralgia, muscle soreness, backache, postoperative, posttraumatic or after tooth extraction pain, inflammation, Sprain pain, bruise pain, toothache, headache, gout pain, algomenorrhea, dysmenorrhea, afterpain, pelvic imflammatory diaease, spine inflammation, urocystitis, anterior segment inflammation and/or acute upper respiratory inflammation (cold, common cold, and pharyngolaryngitis, etc.).

Digestive disturbances and nephropathy by the medicinal composition of this invention are extremely few, in addition, Ornoprostil shows the excellent reductive action on digestive disturbance and nephropathy a little and the side effects such as diarrhea are also extremely few.

### BEST MODE FOR CARRYING OUT THE INVENTION

Though examples of formulation and experiment examples (pharmacological tests, elution tests of formulation and blood kinetics tests) are shown as examples as follows, these are those for understanding the present invention well, and are not for limiting the range of it.

Codes used in each example present the following meanings.
Di = Diclofenac sodium,
In = indomethacin,
Ac = Acemetacin,
OU = Ornoprostil,
Ml = Misoprostol,
GC = glycerol.

### Example 1 of formulation: Soft capsule-1

To 528 g of tricaprilin, 72 g of glyceryl monostearate (MGS-B) was added and heated to about 65°C to dissolve MGS-B. After cooling, 300g of a tricaprilin solution containing Ornoprostil (60 mg/300 g) was added thereto, and mixed with them. In addition, 300 g of Diclofenac sodium was added thereto and stirred, and the residues were crushed by using MYCOLLOIDER and then screened by 180 µm (80 mesh) sieve. The filtrate was made a formula solution by vacuum degassig.

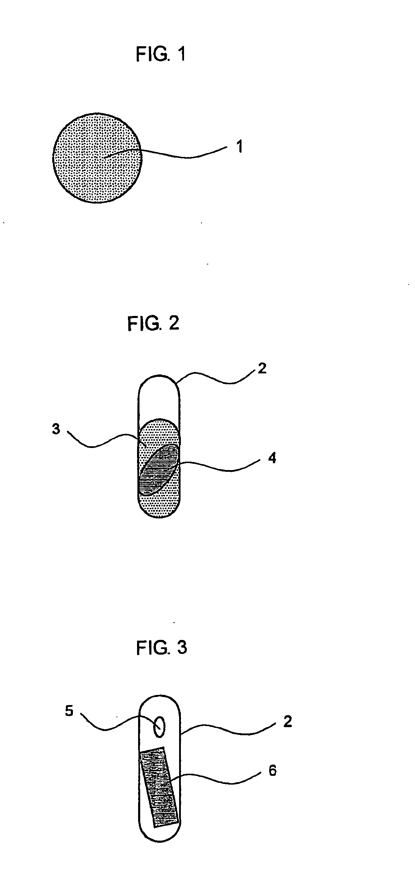

By filling, molding, drying the above prescription solution by using a soft capsule film which contains gelatin, concentrated glycerin, and a D-sorbitol solution by the law of the art, and making them to soft capsules, these soft capsules (12,000 capsules in total) of the following prescription was manufactured. Figure 1 shows composition of these soft capsules. Figure 1 shows a feature in which Di is distributed in an MCT solution of OU.

| Prescription of soft capsule -1 | |
|---|---|
| | |

| Elements of prescription solution | Amounts (mg) |
|---|---|
| Ornoprostil | 0.005 |
| Diclofenac sodium | 25 |
| Glyceryl monostearate | 6 |
| Tricaprilin | 69 |
| Elements of a soft capsule film | |
| Gelatin | 50 |
| Concentrated glycerin | 5 |
| D-sorbitol solution | 15 |
| Total | 170 mg |

### Example 2 of formulation: Soft capsule -2

By using the method described in Example 1 of formulation, soft capsules of the following prescription in which the composition was presented in Figure 1 was manufactured.

| Prescription of soft capsule -2 | |
|---|---|
| | |

| Elements of prescription solution | Amount (mg) |
|---|---|
| Ornoprostil | 0.005 |
| Diclofenac sodium | 25 |
| Glyceryl monostearate | 6 |
| Polysorbate 80 | 3 |
| Tricaprilin | 66 |
| Elements of soft capsule skin | |
| Gelatin | 50 |
| Concentrated glycerin | 5 |
| D-sorbitol solution | 15 |
| Total | 170 mg |

### Example 3 of formulation: Hard capsule -1

By filling a tricaprilin solution containing Ornoprostil (composition 1) and soft capsules containing Diclofenac sodium (composition 2) manufactured by a method described in Example 1 of formulation (however, Ornoprostil is not added) into No.1 gelatin hard capsule by the law of the art, hard capsules of the following prescription, which contained an MCT solution (3) containing OU and an capsule (4) containing Di in a hard capsule (2) as the composition is shown in Figure 2, were manufactured.

| Prescription of hard capsule -1 | |
|---|---|
| | |

| Elements of composition 1 | Amount (mg) |
|---|---|
| Ornoprostil | 0.005 |
| Tricaprilin | 100 |
| Elements of composition 2 | |
| Diclofenac sodium | 25 |
| Glyceryl monostearate | 6 |
| Polysorbate 80 | 3 |
| Tricaprilin | 66 |
| Elements of soft capsule film | |
| Gelatin | 50 |
| Concentrated glycerin | 5 |
| D-sorbitol solution | 15 |
| Total | 270 mg |

### Example 4 of formulation: Hard capsule -2

### 1) Manufacturing method of seamless soft capsules (composition 1 ) containing Omoprostil.

In 34 g of tricaprilin, 50 mg of Ornoprostil was dissolved, and seamless soft capsules (10,000 capsules in total) were prepared by using coating which contains 54 g of gelatin and 6 g of concentrated glycerin by the law of the art.

### 2) Manufacturing method of Diclofenac tablets (composition 2).

Uncoated tablets can be obtained by mixing and compressing 250 g of Diclofenac sodium, 786 g of granulated lactose, 280 g of crystalline cellulose, 70 g of carboxymethyl starch sodium (it is marked with CMS-Na), and 14 g of magnesium stearate.

By using of a coating solution (weight ratio=10:1:1) which contains hydroxypropylmethylcellulose (marked with HPMC), talc, and polyethylene glycol 6000 (marked with PEG6000), Diclofenac tablets (10,000 in total) were obtained by coating 20% of weight of the tablet with a pancoating machine.

### 3) Manufacturing method of gelatin hard capsules.

By filling the above seamless soft capsules (composition 1) and Diclofenac tablets (composition 2) into gelatin hard capsules, hard capsules (10,000 in total), which contained a seamless soft capsule (5) containing OU and a Di tablet (6) in a hard capsule (2) as the composition is shown in Figure 3, were manufactured.

| Prescription of hard capsule -2 | |
|---|---|
| | |

| Elements of composition 1 | Amount (mg) |
|---|---|
| Ornoprostil | 0.005 |
| Tricaprilin | 3.4 |
| Elements of soft capsule film | |
| Gelatin | 5.4 |
| Concentrated glycerin | 0.6 |
| Elements of composition | 2 |
| Diclofenac sodium | 25 |
| Granulated lactose | 78.6 |
| Crystalline cellulose | 28 |
| CMS-Na | 7 |
| Magnesium stearate | 1.4 |
| HPMC | 23.3 |
| Talc | 2.3 |
| PEG6000 | 2.3 |
| Total | 177.3 mg |

### Experiment example 1: Effect of Ornoprostil for NSAID-induced stomach ulcer. (1) Effect for NSAID-induced stomach ulcer (non-inflammatory model).

After weighing Lew/Crj strain maleness rats which were in fasting state for 16-24 hours on free water intake, they were so divided that average weights of each group might almost be equal. The specimen solution (each NSAID suspension and a solution which did not include OU or a solution which includes OU were used together simultaneously (called the OU non-containing solution administered group and the OU containing solution administered group, respectively)) were administered to these rats, they were killed by dislocation of cervical vertebra 4 hours later, and the stomachs were removed. To facilitate observations of gastric mucosa disorder, 3 hours and half later after administering the specimen solution, 0.5 ml of a physiological salt solution of 4% brilliant blue 6B (brilliant blue 6B) was injected from the caudal vein. Removed stomachs were internally filled a physiological salt solution, in addition, soaked in 10% neutral buffered formalin, and fixed. After fixed, they were incised along greater curvature, and after washed in a physiological salt solution lightly, the presence of ulcer development was observed and the length of the ulcer was measured. The inhibition ratio (ratio of length of ulcer in the OU containing solution administered group to that of the ulcer in the OU non-containing solution administered group) of the ulcer was calculated. The following media were used for formulation of the specimen solution.
- Each NSAID suspension: 0.5% methyl cellulose suspension.
- The OU non-containing solution or the OU containing solution: a, phosphate buffer (pH7.4) containing 0.5% ethanol.
   Because as a result of a preliminary examination to set the NSAID dosage (only each NSAID suspension was administered as a specimen solution) Di and Ac were 30 mg/kg and In was 20 mg/kg as dosages wherein the comparable ulcer was caused in each NSAID administered group, these dosage were used in this examination and the examination thereafter.

### (2) Analgesic effect in inflammatory model and an effect on NSAID-induced stomach ulcer.

After weighing Lew/Crj strain maleness rats, 0.1 ml of Mycobacteria Butyricum (Mycobacterium Butyricum) suspended into liquid paraffins per one were administered into intrafoot-pad of left hind-limb under etherization, and were phlogogenic. This day was assumed to be the first phlogogenic treatment day. After weighing on 21 days after phlogogenic treatment, rats, which these right hind-limb of ankle joints were stimulated by winding and stretching 5 times every 4-5 seconds and these bark reactions were shown all of 5 times, were chosen, and so divided that the average weigh of each group could be almost comparable. They were in fasting for 16-24 hours on free water intake from a day before measure. The specimen solutions (each NSAID suspension and the OU non-containing solution or the OU containing solution are used together simultaneously) were orally administered, and bark reactions were observed at intervals of 1 hour from 1 hour to 4 hours after administered. When stimulation like the above was added at each measure and bark reactions were not observed at all among 5 trials, it was judged that bark reactions could be negative. At 4 judges after administered the specimen solutions, it was judged that there were analgesic effects for once or more negative rats, and the efficiency (the number of animals with analgesic effects/that of laboratory animals) was calculated. Moreover, as well as (1), and the inhibition ratio of ulcer was calculated. Table 1 shows the measurement results of (1) and (2).

**Table 1:**

| Antiulcer effects and analgesic effects by NSAID and OU simultaneous administration (Rat oral administration, non-inflammatory model, and inflammatory model). | | | |
|---|---|---|---|
| | (1) Non-inflammatory model | (2) Phlogogenic model | |
| Medicine (dosage) | Inhibition ratio of ulcer (%) | Inhibition ratio of ulcer (%) | Analgesic effect Efficiency (examples) |
| Invention: Di (30 mg/kg) + OU (3 µg/kg) | 67.30 | 45.20 | 8/10 |
| Comparison 1: In (20 mg/kg) + OU (3 µg/kg) | 15.80 | 29.40 | 5/10 |
| Comparison 2: Ac (30 mg/kg) + OU (3 µg/kg) | 37.70 | 0 | 8/10 |

As shown in Table 1, it can be understood that an analgesic effect (principal action) and an antiulcer effect (reduction action for side effects) in the combination of the Di+OU administered group (invention) are stronger than those in the combination of the In+OU administered group (comparison 1) and the Ac+OU administered group (comparison 2), and the combination of them is the best.

Moreover, efficiency of analgesic effect by each NSAID in the OU administered group was equal to that in the OU non-administered group, and the OU administration was not influenced to analgesic effect of NSAID.

### Experiment example 2: Comparison of suppressive effects of Omoprostil or Misoprostol to Diclofenac-induced stomach ulcer.

After weighing Lew/Crj strain maleness rats which were in fasting for 16-24 hours on free water intake, they were so divided that average weight of each group might almost be equal. A specimen solution (30 mg/kg suspension of Di and the OU non-containing solution or the OU containing solution, or the Ml non-containing solution or the Ml containing solution are used together simultaneously) was administered to these rats, they were killed by dislocation of cervical vertebra 4 hours later, and the stomachs were removed. Hereafter, according to Experiment example 1, the inhibition ratio (ratio of length of ulcer in each PG (OU, Ml) non-containing solution administered group to that of ulcer in each PG (OU, Ml) containing solution administered group) was calculated. The following media were used for formulation of the specimen solution.
• Di suspension: 0.5% methyl cellulose suspension.
• Each PG (OU, Ml) non-containing solution or the OU and Ml containing solution: a phosphate buffer (pH7.4) containing 0.5% ethanol.

**Table 2:**

| Suppressor effect of Ornoprostil or Misoprostol to Diclofenac-induced stomach ulcer. | | |
|---|---|---|
| | Medicine (dosage) | Efficiency of Analgesic effect (%) |
| Invention | Di (30 mg/kg) + OU (3 µg/kg) | 67.3 |
| Comparison | Di (30 mg/kg) + Ml (30 µg/kg) | 56.0 |

The antiulcer action of Ornoprostil to the Diclofenac-induced stomach ulcer was 10 times or more higher than that of Misoprostol as shown in Table 2.

### Experiment example 3: Inducing effect of diarrhea (rats and oral administration).

Doses of Ornoprostil or Misoprostol which generate induced effect of diarrhea are 400 µg/kg or more (0/5 examples) and 50, 100 µg/kg (each 0/5 examples and 1/5 examples), respectively, and in the margin of safety (the maximum nontoxic amount), the effect of Omoprostil is at least 8 times higher than that of Misoprostol (values in parentheses mean the number of animals which cause diarrheal/the number of laboratory animals.).

As the result of experiments 1-3 on the above, it is understood that the combination of Di and OU is the best as the combination of NSAID and PG.

### Experiment example 4: Administering times and antiulcer effects of Diclofenac and Ornoprostil.

Suppressive effects of ulcer (the incidence rate of ulcer = the number of animals which cause ulcer / the number of laboratory animals, the inhibition ratio of ulcer) when an administering time of the Ornoprostil solution is assumed to be before administration of the NSAID (Diclofenac sodium) suspension, simultaneously with administration of the NSAID suspension (immediately before administration) or after administration of the NSAID suspension, respectively, were measured. Table 3 shows the result.

**Table 3:**

| Relations between administering time of Ornoprostil and suppressive effect of Diclofenac sodium-induced stomach ulcer (oral administration to rat). | | | |
|---|---|---|---|
| Administering time of OU | Incidence rate of ulcer (Number of Examples) | Inhibition ratio of ulcer (%) | Antiulcer coefficient* |
| 60 minutes before Di administering | 7/8 | 85.1 | 97.3 |
| 30 minutes before Di administering | 7/8 | 49.2 | 56.2 |
| 10 minutes before Di administering | 5/8 | 78.2 | 125.1 |
| 5 minutes before Di administering | 6/8 | 75.7 | 100.9 |
| 0 minutes before Di administering | 7/8 | 76.0 | 86.9 |
| 5 minutes after Di administering | 7/8 | 6.2 | 7.1 |
| 10 minutes after Di administering | 8/8 | 28.8 | 28.8 |
| 30 minutes after Di administering | 7/8 | 14.1 | 16.1 |
| 60 minutes after Di administering | 6/8 | 3.1 | 4.1 |

| | | | |
|---|---|---|---|
| *Antiulcer coefficient=lnhibition ratio of ulcer/Incidence rate of ulcer | | | |

Though OU was effective for suppressor of ulcer when an administering time of OU was simultaneously with administration of Di, or before administration of Di, especially from 10 to 5 minutes ago(the antiulcer coefficient was an index.) from administration of Di, the effect had decreased after administration of Di.

### Experiment example 5: Elution tests of formulations of the present invention.

An elution test (rotation basket method: 250 rpm, eluate: isotonic solution (500 ml) of artificial gastric fluid (0.25% SDS and pH1.4), 37°C) of the active ingredient of the formulation manufactured in Example 1 of the formulation (OU and Di) was done. Table 4 shows the result.

**Table 4:**

| elution rates of active ingredients (OU, Di) in formulations of the present invention in artificial gastric fluid. | | |
|---|---|---|
| Time (minute) | OU elution rate (%) | Di elution rate (%) |
| 0 | 0 | 0 |
| 10 | 42 | 24 |
| 15 | 104 | 40.9 |
| 20 | 98 | 45 |
| 30 | 105 | 50.5 |

From Table 4, it is sufficiently forecast to be eluted Diclofenac in stomach following (in the time difference of 5-10 minutes) elution of Omoprostil after the formulation of the present invention is administered.

### Experiment example 6: Antiinflammatory effect of the formulation of the present invention (blood level of Diclofenac).

Formulation specimens (numbers of specimens which correspond to 75 mg as dosage of Di) containing Di described later was orally administered to female beagles (10-15 months of age and 5 total), which had bred with solid fodder (DS-5 (trade name); Oriental Yeast Co., Ltd., 200 g/time/day) on free water intake, compulsorily at 1 hour after feeing. In addition, immediately later, water (10 ml) was orally administered with stomach by catheter compulsorily. Blood was collected from cephalic vein before administration of the specimen, 0.5, 1, 2, 3, 4, 6, and 24 hours later from the administration. Plasma was obtained by centrifugal separation of the gathered blood (at 4°C, 3000 rpm, for 15 minutes). The concentration of Di in the obtained plasma was measured.

In addition, a similar experimental procedure was done twice (3 times in total) by substituting the formulation specimen containing Di (the following reference) every a week by using each laboratory animal. Cmax (the maximum blood level), Tmax (a time from beginning of dosage until reaching the maximum blood level) and the AUC (the area under the curve) (mean) were calculated from the blood level of measured Di. Table 5 shows the result.

### The formulation specimens containing Di:

• Di+OU mixture: Formulation manufactured in Example 1 of formulation (mixture of Di (25 mg) + OU (5 µg)).
• Single formulations of Di: Voltaren tablets (trade name) (single formulation of Di (25 mg)) (the commercial single formulations).
• Di+Ml mixture: Arthrotec tablets (trade name) (mixture of Di (75 mg) + Ml (200 µg)) (the commercial mixtures).

**Table 5:**

| Antiinflammatory effects of formulation of the present invention (blood level of Diclofenac) (n=5). | | | |
|---|---|---|---|
| Administered formulations (Content of medicine and administered amount per single agent) | Cₘₐₓ (µg/ml) | Tₘₐₓ (hr) | AUC (µg·hr/ml) |
| Administered group of mixture (3 tablets) Di (25 mg) + OU (5 µg) (Invention: Example 1 of formulation) | 14.0 | 1.5 | 52.8 |
| Administered group of Di (25 mg) single formulation (3 tablets) (comparison formulation 1: Voltaren tablets (trade name), the commercial single formulations.) | 11.2 | 5.8 | 52.6 |
| Administered group of mixture (1 tablet) Di (75 mg) + Ml (200 µg) (comparison formulation 2: Arthrotec tablets (trade name), marketed mixture.) | 6.7 | 19.4 | 21.1 |

From Table 5, it is forecast that the formulation of the present invention could possessed more than equal Cmax compared with the comparison formulation 1 and 2 and more than equal effect (antipyretic, paregoric, and antiinflammatory). In addition, it is forecast that the formulation of the present invention could have immediate action because the Tmax is earlier than that of the comparison formulation 1 and 2. Moreover, it was understood that invention formulation has an AUC, which is almost equal to the comparison formulation 1.

As the above, it is understood that the compounding ingredients of Diclofenac and Omoprostil of the present invention could have an effect (antipyretic, paregoric, and antiinflammatory effect) and immediate action equally to or more than the single formulation of Diclofenac on the market and the mixture of Diclofenac and Misoprostol on the market.

### Experiment example 7: Reduction effects to side effects of formulations of this invention (stomach ulcer, diarrhea, and vomit).

After weighing beagles (7-8 months of age) bred on free water intake, they were so divided that average weight of each group might almost be equal (2-3 males and females in 5 beagles per each group.). The above formulation specimens containing Di (numbers of specimens which correspond to 225 mg as dosage of Di) was orally administered to the laboratory animals compulsorily. In addition, immediately later, water (10 ml) was orally administered by gastric catheter compulsorily. After anesthetizing by intravenous administration of pentobarbital (25 mg/kg), following be laid, at 24 hours ago from specimen administering and 24 hours later, the video scope for the animal (VQ-7112A (trade name), Olympus optical Co., Ltd.) was inserted in the stomach and the megascopic findings (the festering level of the gastric mucosa was observed.) was recorded, then the antropyloric gastric mucosa was taken picture. Moreover, 10 ml of 4% brilliant blue was administered into intravenous, the antropyloric gastric mucosa was taken picture to observe findings following injection of coloring matter, and the level of the stomach ulcer was judged from this picture. Moreover, the presence of appearance of diarrhea and vomit within 24 hours after administering the specimen was observed.

Table 6 shows the result.

**Table 6:**

| Reduction effects to side effects of formulation of this invention (stomach ulcer, diarrhea, and vomit). | | | |
|---|---|---|---|
| Administered formulations (Content of medicine and administered amount per single agent) | Ulcer Incidence | Diarrhea Incidence | Vomit Incidence |
| Administered group of Di (25 mg) + OU (5 µg) mixture (9 tablets) (Invention: example 1 of formulation) | 1/5 | 0/5 | 0/5 |
| Administered group of Di (25 mg) single formation (9 tablets) (Comparison formulation 1: Voltaren tablets (trade name), the commercial single formulation.) | 5/5 | 0/5 | 0/5 |
| Administered group of Di (75 mg) + Ml (200 µg) mixture (3 tablets) (Comparison formulation 2: Arthrotec tablets (trade name) and the commercial mixture.) | 5/5 | 4/5 | 2/5 |
| (both, number of generation/number of animal.) | | | |

From Table 6, the incidence of stomach ulcer in administered group of the formation of the present invention was 1/5 (the extent of stomach ulcer (only 1 example) which occurred in the administered group of this invention was also slighter than the administered group of comparison formation 1 and 2.) to that in the comparison formation 1 and 2 administered groups, and neither vomit nor diarrhea have been completely generated. Therefore, it is understood that the formulation of the present invention is an antipyretic drug, a paregoric drug, and antiphlogistine in which the side effects were greatly reduced.

### Experiment example 8: Effect of Ornoprostil on nephropathy.

After weighing SPF/Crj strain maleness rats (9 weeks of ages), they were so divided that average weight of each group might almost be equal. At 48 hours after 50% glycerol (10 ml/kg) (medium: physiological salt solution) was subcutaneously administered to rats deprived water for 48 hours (± 2 hour) on feeing, about 1.5 ml of blood was gathered from the jugular under ether anesthesia. Ornoprostil (medium: 0.4% Tween 80 solution containing 1% dehydrated ethanol) was orally administered immediately before administration of glycerol. Water was supplied to them 2 hour later from administration of glycerol. Those serums were obtained by centrifugal separation (for about 4°C, 3000 rpm, for 15 minutes) of the gathered blood. Urea nitrogen (abbreviated as UN.) (urease•GIDH method) and creatinine (abbreviated as CRE.) (Creatininase•F-DAOS method) in the obtained serum were measured with the biochemistry automated analyzer (AU400 (trade name), Olympus Optical Co., Ltd.). Table 7 shows the result.

**Table 7:**

| Suppressive effect of Ornoprostil to induced nephropathy. | | | |
|---|---|---|---|
| Test groups | Dosage | UN (mg/dL) | CRE (mg/dL) |
| Control Phlogogenic Invention | Medium* (subcutis) + medium** | 18.8 | 0.19 |
| | GC (subcutis) + medium** | 163.7 | 2.74 |
| | GC (subcutis) + OU (3 µg) | 89.7 | 1.38 |
| | GC (subcutis) + OU (10 µg) | 79.9 | 1.03 |

| | | | |
|---|---|---|---|
| *: physiological salt solution, | | | |
| **: 0.4% Tween 80 solution containing 1% ethanol. | | | |

From Table 7, because Ornoprostil depressed the glycerol-induced nephropathy (rise of UN value and CRE value in serum), which is ischemic renal failure model, it was understood that it could be effective to nephropathy. Moreover, the amount of dosage is equal to that of the dosage which showed the suppressive effect of stomach ulcer accompanying NSAID presented by Experiment example 1. Therefore, it is forecast that the mixture of Ornoprostil and Diclofenac could reduce the aggravation of nephropathy that Diclofenac possesses. The suppressive effect of nephropathy is an effect that not admitted in the acid secretion inhibitor and/or the protection factor promoter.

### EFFECT OF THE INVENTION

From the above data, it is evident that the medicinal composition of the present invention, which combines Diclofenac or the nontoxic salt with Ornoprostil, could excel as NSAID in the drug action and the immediate effect of the analgesic effect, etc. and the side effects (gastrointestinal ulcer, nephropathy, and diarrhoea/vomitus action) of NSAID and PG could be little.

Specific effects of medicine compositions of the present invention are as follows.
1) Comparing the combination with NSAID (Di, In, Ac) to that of the combined medication with OU, the Di+OU administered group (invention) is the best in the suppressive effect of stomach ulcer and the combined medication with OU did not influence the analgesic effect of NSAID (Di is contained.) (Experiment example 1, Table 1).
2) The utility of the Di+OU administered group (invention) was 80 or more times more excellent than that of the Di+Ml administered group (10 or more times suppressive action (Experiment example 2, Table 2) of ulcer and 8 or more times margin of safety, 1/8 or less induced effect of diarrhea (Experiment example 3)).
3) The ulcer suppressive effect of OU to Di was effective simultaneously with administration of Di or before administration of Di, especially at from 5 to 10 minutes ago before administration of Di (Experiment example 4, Table 3). Moreover, it was confirmed that the compounding ingredients of the present invention could agreed with the experimental results of this combined medication (Experiment example 5, Table 4).
4) Comparing each blood level of Di when the compounding ingredients of the present invention (the Di+OU compounding ingredient), commercial Voltaren tablets (trade name) (Di single agent), and commercial Arthrotec tablets (trade name) (Di+Ml compounding ingredient) were administered to dogs, increase of Cmax and shortening of Tmax by the compounding ingredients of the present invention was admitted compared with the other two formulations on the market (Experiment example 6, Table 5). For the compounding ingredients of the present invention, reinforcement and fast-acting property in the effect (paregoric, antipyretic, and antiinflammatory effect) of Diclofenac can be expected compared with two formulations on the market.
5) Because induction of stomach ulcer and development of diarrhea/vomit by the compounding ingredients of the present invention is fewer than the other two formulations on the market, as a result of comparing side effects (induction of stomach ulcer and development of diarrhea/vomit) when the compounding ingredients of this invention, Voltaren tablets (trade name), and Arthrotec tablets (trade name) were administered to a dog, it was understood that the compounding ingredients of the present invention could be useful formulations (Experiment example 7, Table 6).
   Moreover,
6) It proved that Ornoprostil could be effective to treatment and prevention of nephropathy by the same dosage to suppress Diclofenac-induced stomach ulcer (Experiment example 8, Table 7). Therefore, it is expected that the combination of OU and Di of the present invention could reduce progression of nephropathy, which is the side effect that Diclofenac possesses.

## Claims

1. A medicinal composition comprising Diclofenac or its nontoxic salt and Ornoprostil.

2. The medicinal composition of claim 1, which is an antipyretic, an analgesic drug and/or an antiphlogistine.

3. The medicinal composition of claim 2, which is a medicine and/or prophylactic drug for rheumatoid arthritis, rheumatic fever, osteoarthritis, ankylosing spondylitis, spondylopathy, periarthritis, cervico-omo-brachial syndrome, tendon marrow inflammation, neuralgia, arthralgia, muscle soreness, backache, postoperative, posttraumatic or after tooth extraction pain, inflammation, Sprain pain, bruise pain, toothache, headache, gout pain, algomenorrhea, dysmenorrhea, afterpain, pelvic imflammatory diaease, spine inflammation, urocystitis, anterior segment inflammation and/or acute upper respiratory inflammation.

4. The medicinal composition of claim 2 or 3, which can reduce digestive disturbance induced by Diclofenac or the nontoxic salt.

5. The medicinal composition of claim 4, which can reduce digestive disturbance induced by Diclofenac or the nontoxic salt.

6. A formulation of the medicinal composition of claim 1, wherein Ornoprostil is discharged simultaneously with or before discharge of Diclofenac or the nontoxic salt in gastrointestinal tract.

7. The formulation of claim 6, which contains Diclofenac or the nontoxic salt, Ornoprostil, and a stabilizer of Ornoprostil.

8. The formulation of claim 7, which contains a suspending agent.

9. The formulation of any one of claims 6 to 8, which is a soft capsule mixed Diclofenac with Omoprostil.

10. The formulation of claim 6 or 7, which is a tablet mixed Ornoprostil with Diclofenac.

11. The formulation of claim 6 or 7, which is a tablet coated with Ornoprostil to a nucleus made from Diclofenac.

12. The formulation of claim 6 or 7, which is a hard capsule comprising a soft capsule containing Diclofenac and a solution containing Ornoprostil.

13. The formulation of claim 6 or 7, which is a hard capsule comprising a granule, a capsule or a tablet containing Ornoprostil and a granule, a capsule or a tablet containing Diclofenac.

14. The formulation of claim 7, wherein the stabilizer of Ornoprostil is medium chain triglyceride (MCT).

15. The formulation of claim 14, which is a capsule comprising an MCT solution in which Ornoprostil has been dissolved and Diclofenac or the nontoxic salt has been scattered.

16. The formulation of claim 14, which is a hard capsule comprising a tablet or a capsule which contains an MCT solution containing Ornoprostil and Diclofenac or the nontoxic salt.

17. The formulation of claim 14, which is a hard capsule comprising a capsule which contains an MCT solution containing Ornoprostil and a tablet or a capsule which contains Diclofenac or the nontoxic salt.

18. The formulation of claim 14, which is a hard capsule comprising a capsule which contains an MCT solution containing Ornoprostil and a tablet of Diclofenac or the nontoxic salt, which is covered with coating containing a water-soluble polymer.

19. The formulation of claim 18, wherein the water-soluble polymer is hydroxypropylmethylcellulose.

20. The formulation of any one of claims 14 to 18, wherein MCT is tricaprilin.

21. A method of reducing digestive disturbance and/or nephropathy induced by Diclofenac or its nontoxic salt, which comprises administering a medicinal composition comprising Diclofenac or its nontoxic salt and Ornoprostil.

22. The method of reducing digestive disturbance and/or nephropathy induced by Diclofenac or the nontoxic salt of claim 21, wherein Ornoprostil is administered simultaneously with or before administration of Diclofenac or the nontoxic salt.

23. A prophylactic drug and/or medicine for nephropathy, which comprises Ornoprostil as an active ingredient.

24. A prophylactic and/or therapeutic method for nephropathy, which comprises administering Ornoprostil.
